# EUROPEAN PATENT SPECIFICATION

(11) **EP 1 329 460 B1**
(45) Date of publication and mention of the grant of the patent: **28.06.2006**
(21) Application number: 01978881.9
(22) Date of filing: 24.10.2001
(51) Int. Cl.: C07K 14/765, C07K 1/14, C12P 21/02

(54) **METHOD OF MONOMERIZING HUMAN SERUM ALBUMIN POLYMERS**
VERFAHREN ZUR MONOMERISIERUNG VON HUMANEN SERUMALBUMIN-POLYMEREN
PROCEDE DE MONOMERISATION DE POLYMERES D'ALBUMINE SERIQUE HUMAINE

(30) Priority: 24.10.2000 JP 2000324027; 24.10.2000 JP 2000324028
(43) Date of publication of application: 23.07.2003
(73) Proprietor: Juridical Foundation, The Chemo-Sero-Therapeutic Research Institute, Kumamoto-shi, Kumamoto 860-8568 (JP)
(72) Inventor: Nouchi, Toshinobu c/o Kikuchi Research Center, Kumamoto 869-1298 (JP); Mizokami, Hiroshi c/o Kikuchi Research Center, Kumamoto 869-1298 (JP); Tajima, Yoshitaka c/o Kikuchi Research Center, Kumamoto 869-1298 (JP); Yokote, Hiroyuki c/o Kikuchi Research Center, Kumamoto 869-1298 (JP); Adachi, Satoshi c/o Kikuchi Research Center, Kumamoto 869-1298 (JP); Miyatsu, Yoshinobu c/o Kikuchi Research Center, Kumamoto 869-1298 (JP); Tanabe, Tetsuro c/o Kikuchi Researh Center, Kumamoto 869-1298 (JP); Ushio, Yoshitaka c/o Kikuchi Research Center, Kumamoto 869-1298 (JP); Shibata, Shinichi c/o Kikuchi Research Center, Kumamoto 869-1298 (JP)
(74) Representative: Geering, Keith Edwin
(86) International application number: PCT/JP2001/009334
(87) International publication number: WO 2002/034785

(56) References cited:
- EP-A- 0 420 007
- EP-A- 1 329 461
- EP-A- 1 329 462
- EP-A1- 0 319 067
- EP-A2- 0 570 916
- US-A- 4 169 829
- US-A- 5 986 062
- GIANAZZA ELISABETTA ET AL: "Structural transitions of human serum albumin: An investigation using electrophoretic techniques" ELECTROPHORESIS, vol. 18, no. 5, 1997, pages 695-700, XP009031959 ISSN: 0173-0835
- BURTON S J ET AL: "REFOLDING HUMAN SERUM ALBUMIN AT RELATIVELY HIGH PROTEIN CONCENTRATION" EUROPEAN JOURNAL OF BIOCHEMISTRY, vol. 179, no. 2, 1989, pages 379-388, XP009031960 ISSN: 0014-2956
- HORI J: "ALKALINE DENATURATION OF BOVINE SERUM ALBUMIN II. ANALYSIS BY ISOELECTRIC FOCUSING IN POLYACRYLAMIDE GELS" JAPANESE JOURNAL OF ELECTROPHORESIS, vol. 31, no. 6, 1987, pages 401-408, XP009031961 ISSN: 0031-9082
- LEONARD JR, W J ET AL: "A structural transformation in bovine and human plasma albumins in alkaline solution as revealed by rotatory dispersion studies" THE JOURNAL OF BIOLOGICAL CHEMISTRY, vol. 238, no. 6, 6 June 1963 (1963-06-06), pages 1984-1988, XP002283840

## Description

### Technical Field

The present invention relates to a method for converting a multimer of human serum albumin into monomers thereof. More specifically, the present invention pertains to a method for converting, into monomers, a multimer of human serum albumin, which is generated during the production of human serum albumin from the human plasma as a raw material therefor or during the production of human serum albumin according to the gene recombination technique, the method comprising the step of treating the multimer with an alkaline solution (hereunder also referred to as "alkali-treatment").

The present invention also relates to a method for preventing the uncorrected folding of human serum albumin formed through the foregoing treatment with an alkaline solution, which is caused due to the formation of incorrect inter-molecular or intra-molecular disulfide bonds, the method comprising the step of addition of an SH group-pataining compound.

### Background Art

Human serum albumin (hereunder also referred to as "HSA") is a principal protein component present in the plasma, consists of a single chain polypeptide comprising 585 amino acid residues and has a molecular weight equal to about 66,000 Dalton (see Minghetti, P. P. et aL (1986), Molecular structure of the human albumin gene is revealed by nucleotide sequence within 11-22 of chromosome 4. J. Biol. Chem. 261, pp. 6747-6757). It has been known that the principal roles of HSA are not only to maintain the normal osmotic pressure of the blood, but also to bind with a variety of substances such as calcium ions, fatty acids, bilirubin, tryptophan and drugs possibly present in the blood, thereby playing a role of a carrier for transporting these substances. Purified HSA has been used in, for instance, the postoperative treatment after surgical operations and the treatment of hypoalbuminemia caused due to the loss of albumin such as hemorrhagic shock, burn and nephrotic syndromes.

Conventionally, HSA has been prepared by subjecting the human plasma to the low temperature ethanol-fractionation method of Cone or any method similar thereto to give HSA-containing fractions (HSA is fractionated in the fraction V) and then purifying the fraction through the use of a variety of purification techniques. Moreover, there has recently been developed a method in which the human plasma is not used as a raw material, for instance, a technique for producing human serum albumin using yeast, *Escherichia coli* or *Bacillus subtilis* cells, while making use of the gene recombination technique.

These gene recombination techniques are detailed in (1) Production of Recombinant Human Serum Albumin from *Saccharomyces cerevisiae*; Quirk, R. et al. Biotechnology and Applied Biochemistry, 1989, 11: 273-287, (2) Secretory Expression of the Human Serum Albumin Gene in the Yeast, *Saccharomyces cerevisiae,* Ken Okabayashi et al. J. Biochemistry, 1991, 110: 103-110, (3) Yeast Systems for the Commercial Production of Heterologous Proteins; Richard G. Buckholz and Martin A. G. Gleeson, Bio/Technology, 1991, 9: 1067-1072 for the yeast, (4) Construction of DNA sequences and their use for microbial production of proteins, in particular, human serum albumin; Lawn, R. M. European Patent Publication No. 0073646A (1983), (5) Synthesis and Purification of mature human serum albumin from *E. coli*; Latta, L. et al. Biotechnique, 1897, 5: 1309-1314 for the *Escherichia coli (E. coli)*, (6) Secretion of human serum albumin from *Bacillus subtilis*; Saunders, C. W. et al. J. Bacteriol. 1987, 169: 2917-2925 for the *Bacillus subtilis.*

The methods for purifying the human serum albumin usable herein in general include those currently used in the protein chemistry such as a salting out method, an ultrafiltration method, an isoelectric precipitation method, an electrophoresis method, an ion-exchange chromatography technique, a gel filtration chromatography technique and/or an affinity chromatography technique. Indeed, the human serum albumin-containing fraction includes various kinds of contaminants originated from, for instance, biological tissues, cells and blood and therefore, the human serum albumin has been purified by a complicated combination of the foregoing methods.

In the industrial production of human serum albumin, it is inevitable to treat the same under various conditions different from environmental conditions observed in the human body and accordingly, multimers of human serum albumin are formed. There has not yet been known any such a report that these multimers adversely affect the human body in the clinical application of human serum albumin, but there is such a suspicion that these multimers may develop a novel antigenicity. For this reason, an upper limit in the contamination with these multimers is prescribed in the standardization test of "human serum albumin" as a pharmaceutical agent from the viewpoint of the safety thereof as a medicine and therefore, it becomes an important problem, in the production of a pharmaceutical preparation containing the same, to substantially reduce the content of such multimers in the preparation.

There have been reported several methods for removing the multimers generated during the process for the production of human serum albumin. For instance, Journal of Applied Biochemistry, 1983, 5: 282-292 discloses that HSA having a purity of 99% and an aggregate (synonymous with "multimer") content of not more than 1% was obtained by subjecting the fraction V, prepared according to the ethanol-fractionation of the plasma, to a combination of a variety of chromatography techniques (such as Sephadex G-25, DEAE- and CE-Sepharose CL-6B and Sephacryl S-200); and Japanese Patent Application Serial No. Sho 63-265025 and Japanese Un-Examined Patent Publication No. Hei 2-111728 disclose a method comprising the steps of adding a stabilizer to the fraction V, heat-treating the resulting mixture (at a temperature ranging from 50 to 70°C for 1 to 10 hours) and then subjecting the mixture to the ammonium sulfate precipitation technique, the polyethylene glycol fractionation technique or the isoelectric precipitation technique to thus remove any contaminant and multimer of HSA.

All of these methods relate to methods for preparing HSA free of any multimer thereof, but the methods comprise the step of removing such multimers of HSA generated during the process for the production of the same from a solution containing the multimers. For this reason, the foregoing methods inevitably suffer from a reduction in the yield of HSA monomers since they remove and dispose the multimers of HSA capable of being converted into the monomers thereof and they likewise suffer from a decrease in the yield of monomers accompanied by the foregoing procedures for removing the multimers. The HSA-containing pharmaceutical preparation is one, which is administered to a patient in a large amount and accordingly, should be supplied in a substantially greater amount as compared with other protein-containing pharmaceutical preparations. Consequently, there has been desired for the development of a method for preparing HSA whose multimer content is reduced to a level as low as possible in a higher yield, from the industrial standpoint.

### Disclosure of the Invention

Accordingly, a first object of the present invention is to provide a method for preparing human serum albumin, which permits the efficient conversion of the multimers of human serum albumin that are removed and disposed in the conventional methods, into the monomers thereof.

It is a second object of the present invention to provide a method for preventing any intra-molecular uncorrected folding of a human serum albumin molecule or any inter-molecular uncorrected folding between human serum albumin molecules or between a human serum albumin molecule and other contaminants, formed during a treatment with an alkaline solution (including, for instance, chromatography) in the process for preparing the human serum albumin.

It is a third object of the present invention to provide a human serum albumin product having a high safety as a medicine.

The inventors of this invention have conducted various studies, while taking into consideration the foregoing present status of the art and have found that if an aqueous multimer-containing HSA or rHSA (gene recombinant HSA) solution is allowed to stand over an appropriate period of time under an alkaline condition, the multimers can be converted into the monomers of HSA. The inventors have further found that in the foregoing treatment with an alkaline solution, the addition of an SH group-containing compound such as cysteine to the processing liquid would permit the inhibition of the occurrence of any intra-molecular and/or inter-molecular uncorrected folding of HSA.

The present invention has been completed on the basis of the foregoing findings.

According to a first aspect of the present invention, there is provided a method for converting a multimer of human serum albumin into monomers thereof wherein the multimer is treated with an alkaline solution ofpH 8 to 9.5.

According to a second aspect of the present invention, there is provided a method for converting a multimer of human serum albumin into monomers thereof wherein the multimer is treated with an alkaline solution of pH 8 to 9.5 in the presence of an SH group-containing compound.

According to a third aspect of the present invention, there is provided a method for preventing the uncorrected folding of human serum albumin during a process for converting a multimer of human serum albumin into monomers thereof by treating the multimer with an alkaline solution ofpH 8 to 9.5 wherein the human serum albumin-containing solution is treated with the alkaline solution in the presence of an SH group-containing compound.

According to a fourth aspect of the present invention, there is provided an HSA product whose multimer content is reduced to a level as low as possible and which is prepared by a method for converting the multimer of HSA into the monomers thereof according to the first aspect of the present invention.

According to a fifth aspect of the present invention, there is provided an HSA product free of any such complex and which is prepared by a method according to the second or third aspect of the present invention.

### Brief Description of the Drawings

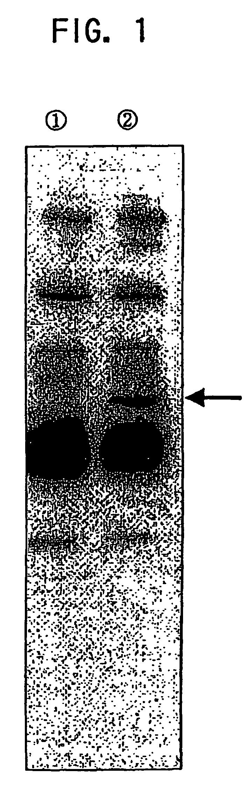

Fig. 1 is a photograph showing the results obtained by subjecting, to the SDS-PAGE, samples of a human serum albumin-containing solution prepared in Preparation Example 1 before and after the treatment with an alkaline solution and then analyzing the samples according to the Western blot technique. In this photograph, the result ① corresponds to the sample before the treatment with an alkaline solution and the result ② corresponds to the sample after the treatment.

### Best Mode for Carrying Out the Invention

The method according to the first aspect of the present invention is characterized in that it comprises the steps of, for instance, adding an alkaline substance to an aqueous solution containing multimers of human serum albumin with stirring to thus alkalize the aqueous solution and then allowing the alkaline aqueous solution containing the multimers to stand for a certain period of time to thus convert the multimers into the monomers of the human serum albumin. This method can appropriately be put into practice once or several times to thus efficiently produce high purity human serum albumin whose multimer content is reduced to a level as low as possible.

The method according to the second aspect of the present invention is characterized in that it comprises the steps of making an SH group-containing compound coexist in the alkaline solution used for converting the multimer of human serum albumin into the monomers thereof in the process for preparing the human serum albumin.

In general, if a protein solution is alkalized, the SH groups present in a protein molecule may sometimes form an inter-molecular or intra-molecular disulfide bond (including disulfide bond-exchange reactions). At this stage, incorrect disulfide bonds are often formed and this may results in the formation of a protein having a three-dimensional structure different from that of the original protein. The formation of this incorrect disulfide bond may occur inter-molecularly or intra-molecularly. Therefore, if a variety of contaminants coexist, such disulfide bonds may be formed between the protein molecule and the contaminants to thus form novel substances. The formation of such incorrect disulfide bond is referred to as "uncorrected folding". If a novel substance is once formed due to this uncorrected folding, it is quite difficult and troublesome to remove the same. The novel substance may cause unfavorable side effects in the patient who receives the administration of the substance such as allergic reactions due to the expression of a novel immunogenicity and other causes. However, the second method of the present invention would permit the effective inhibition of the occurrence of any intra-molecular or inter-molecular uncorrected folding of human serum albumin, which may be caused when alkalizing a human serum albumin-containing solution, and in turn permits the production of human serum albumin having a higher purity.

Sources of multimers of human serum albumin usable in the present invention are not limited to specific ones and may be, for instance, those produced from the HSA derived from the plasma or the HSA produced by the gene recombination technique (hereunder also referred to as "rHSA"), which can be subjected to the alkali treatment of the present invention. The multimers of HSA and rHSA will hereunder simply be referred to as "multimer(s)".

When preparing HSA from the plasma, it would be predicted that the multimer might be formed in each step, but aqueous multimer-containing solutions obtained in any such step may be used in the invention. Examples of such aqueous solution are an aqueous solution of the fraction V obtained through the low temperature alcohol-fractionation and multimer-containing aqueous solutions generated in the subsequent various purification steps (such as chromatography and heat-treatment) of the fraction V.

The multimer-containing aqueous solutions generated in the various steps for preparing rHSA can likewise be used in the present invention. Specific examples thereof are culture broth of rHSA-producing host cells and multimer-containing aqueous solutions generated in the subsequent various purification steps (such as chromatography and heat-treatment) for the culture broth. In this respect, the term "culture broth" used herein includes the culture broth in which the foregoing host cells are cultivated, and crushed host cell-containing liquids wherein the host cells are crushed by any currently used method.

The host cells used for the production of rHSA are not restricted to specific ones inasmuch as they can produce rHSA and examples thereof include yeast cells, bacterial cells, animal cells and plant cells, with yeast cells being preferably used herein.

When the multimers present in an HSA or rHSA-containing solution are treated with an alkaline solution in the process for the production of human serum albumin, the concentration of HSA or rHSA is not limited to any specific one insofar as it is in the dissolved state, but the concentration thereof is preferably not more than 100 mg/ml.

The pH value of the alkaline solution used for converting the multimers of HSA or rHSA into the monomers thereof ranges from 8 to 9.5, more preferably 8.5 to 9.5 and most preferably 9.0.

Chemical substances used for the alkalization of the pH value of the liquid used for the alkali-treatment are not restricted to specific ones. Examples thereof include one or at least two members selected from the group consisting of alkaline organic compounds and alkaline inorganic compounds. Specific examples thereof are ammonia, ammonium salts, basic metal hydroxides (such as sodium hydroxide and potassium hydroxide), borates, phosphates, acetates, oxalates, citrates, tris-hydroxyaminomethane and mixtures of at least two of these substances.

Such chemical substances are used in a concentration, which never causes any modification of HSA or rHSA and which may vary depending on the concentration of the multimer-containing aqueous solution.

The multimers of human serum albumin is converted into the monomers thereof by alkalization of the aqueous multimer-containing solution and then allowing the solution to stand over a predetermined period of time, preferably not less than 15 minutes and more preferably not less than 3 hours. In this method, the time required for allowing the solution to stand does not have any particular upper limit.

The temperature of this alkali-treatment is not likewise be restricted to any specific one inasmuch as it never causes any modification or denaturation of HSA and rHSA and it ranges, for instance, from 0 to 65°C, preferably 10 to 40°C and more preferably room temperature (about 25°C).

The SH group-containing compounds to be added to the HSA or rHSA solution upon the alkali-treatment thereof are not restricted to particular ones inasmuch as they are compounds each having an SH group, but preferred are low molecular compounds each having an SH group. Specific examples thereof include cysteine, cysteamine, cystamine and methionine, with cysteine being preferably used herein. The SH group-containing compound is added to the HSA or rHSA solution in a final concentration preferably ranging from 0.1 to 50 mM, more preferably 0.2 to 15 mM and most preferably 0.5 to 5 mM, with respect to the HSA or rHSA concentration ranging from 1 to 100 mg/ml.

In case of the production of human serum albumin according to the gene recombination technique, a culture broth in which rHSA-secreting host cells are cultivated or a crushed host cell-containing liquid obtained immediately after crushing of the rHSA-producing host cells is centrifuged at a low spin rate and then rHSA in the centrifuged product is highly purified by a variety of purification methods such as a cation-exchange, anion-exchange, gel filtration, salting out, chelate chromatography, hydrophobic chromatography or adsorption chromatography technique or any combination thereof.

Alternatively, the HSA derived from the plasma is purified by, for instance, subjecting the human plasma to low temperature ethanol-fractionation to give a fraction V containing about 90% of HSA, and then treating the fraction V while making use of the foregoing purification methods. The plasma is sometimes subjected to a heat-treatment prior to the low temperature ethanol-fractionation in order to prevent any decomposition by the action of a protease.

The first method of the present invention may be used in any step of the foregoing process for preparing HSA or rHSA. Preferably, it is effective to treat the multimers thereof generated after the step for treating a human serum albumin solution at a pH value of not more than 5, for instance, cation-exchange chromatography and/or anion-exchange chromatography steps, to thus convert them into the monomers of human serum albumin.

The second method of the present invention may likewise be used in any step of the foregoing process for preparing HSA or rHSA. Preferably, it is effective to use the second method when treating, with an alkaline solution, the multimers generated after the step for treating a human serum albumin solution at a pH value of not more than 5, for instance, cation-exchange chromatography and/or anion-exchange chromatography steps to thus convert them into the monomers of the human serum albumin.

### Examples

### Preparation Example 1: Preparation of a Solution of Multimer-Containing Human SerumAlbumin

According to the method disclosed in TOKUHYO Hei 11-509525, rHSA was produced using yeast cells (*Saccharomyces cerevisiae*). This rHSA-containing culture broth was diluted with purified water to a total volume of about two times that of the original one and then the pH value of the diluted solution was adjusted to 4.5 using an aqueous acetic acid solution. Then the solution was loaded onto STREAMLINE SP Column (available from Amersham Pharmacia Biotech Company; diameter 60 cm × 16 cm), which had been equilibrated with a 50 mM sodium acetate buffer solution (pH 4.5) containing 50 mM sodium chloride. Thereafter, the column was washed with a buffer solution identical to that used for equilibrating the column, followed by passing, through the column, a 50 mM phosphate buffer solution (pH 9.0) containing 300 mM sodium chloride to give rHSA-containing fractions.

### Example 1: Conversion of rHSA Multimers into Monomers Using Borate

Tb 10 ml of a 10% rHSA aqueous solution containing 14.90% of multimers prepared according to the procedures used in Preparation Example 1, there was added 15 ml of a 5% (w/v) dipotassium tetraborate and the final concentration of the latter was adjusted to 3% (pH about 9.0). Subsequently, the resulting solution was allowed to stand at room temperature for 3 hours, while collecting samples at appropriate intervals and then subjected to high performance liquid chromatography using a gel filtration column: TSKgel G3000SW (available from Tosoh Corporation), which had been equilibrated with a 0.1 M KH₂PO₄/0.3 M NaCl buffer solution. Then the content of the albumin multimers present in the solution was calculated on the basis of the results thus obtained. The conversion of the multimers into the monomers proceeded along the alkali-treatment over 3 hours. This clearly demonstrates that this method is quite effective for the conversion of the rHSA multimers into the monomers thereof (see Table 1 given below).

**Table 1**

| | 0 hr | 1 hr | 2 hr | 3 hr |
|---|---|---|---|---|
| Monomers (%) | 85.10 | 96.00 | 96.50 | 97.00 |
| Multimers (%) | 14.90 | 4.00 | 3.50 | 3.00 |

### Example 2: Conversion of rHSA Multimers into Monomers Using 0.5 M Sodium Hydroxide Solution

To 50 ml of a 10% rHSA aqueous solution containing 26.10% multimers prepared according to the procedures used in Preparation Example 1, there was added 2.8 ml of a 0.5 M aqueous sodium hydroxide solution and then the pH value of the mixture was adjusted to about 9. Subsequently, the resulting solution was allowed to stand at room temperature for 3 hours, while collecting samples at appropriate intervals and then subjected to high performance liquid chromatography using a gel filtration column: TSKgel 3000SW (available from Tosoh Corporation), which had been equilibrated with a 0.1 M KH₂PO₄/0.3 M NaCl buffer solution. Then the content of the albumin multimers present in the solution was calculated on the basis of the results thus obtained. The conversion of the multimera into the monomers proceeded along the alkali-treatment over 5 hours. This clearly demonstrates that this method is quite effective for the conversion of the rHSA multimers into the monomers thereof (see Table 2 given below).

**Table 2**

| | 0 hr | 0.25 hr | 1 hr | 2 hr | 3 hr | 4 hr | 5 hr |
|---|---|---|---|---|---|---|---|
| Monomer (%) | 73.90 | 84.70 | 85.00 | 86.50 | 86.60 | 86.80 | 87.00 |
| Multimer (%) | 26.10 | 15.30 | 15.00 | 13.50 | 13.40 | 13.20 | 13.00 |

### Example 3

To 50 ml of the rHSA-containing fraction prepared according to the procedures used in Preparation Example 1, there were added cysteine to a final concentration ranging from 0 to 15 mM and 2.8 ml of a 0.5 N sodium hydroxide solution for controlling the pH of the resulting solution to about 9.0, followed by allowing the solution to stand at room temperature for 5 hours. Then an aqueous acetic acid solution was added to the solution to adjust the pH value thereof to 7.0 and to complete the alkaline solution treatment. Then the solution was subjected to SDS-PAGE (10-20% gradient gel) and Western blotting according to the usual methods. Thereafter, goat anti-human serum albumin and anti-goat IgG-HRP conjugate were used as a primary antibody and secondary antibody, respectively and the products separated through the electrophoresis were treated by the chemiluminescence technique to thus emit light, transferred to a film and then developed. Fig. 1 shows the results obtained by analyzing the samples of the human serum albumin-containing solution free of any added cysteine according to the Western blot technique before and after the treatment with an alkaline solution. In the sample ②, which had been treated with an alkaline solution, there was observed a band ascribed to intra-molecular uncorrected folding of rHSA, at a molecular weight of about 68,000. This band observed for each sample obtained after treating with an alkaline solution in the presence of cysteine in a variety of concentrations was stained with Coomassie and the density of each band was determined using software for analysis (Collage Ver. 3). The results thus obtained are summarized in the following Table 3. Each numerical value represents a value relative to the density observed for a sample free of any cysteine, which is defined to be 100%

**Table 3**

| Cysteine Conc. (mM) | Rate of Multimers Formed (Band Density Ratio) |
|---|---|
| 0 | 100 |
| 0.2 | 15 |
| 1 | 4 |
| 5 | 4 |
| 15 | 0 |

The results listed in Table 3 clearly indicate that the intra-molecular uncorrected folding of human serum albumin can effectively be controlled by carrying out the alkali-treatment in the coexistence of cysteine.

According to the first method of the present invention, the quite simple and cost-saving alkali-treatment would permit the efficient conversion of human serum albumin multimers, which have been removed and discarded in the conventional techniques, into monomers thereof. As a result, the content of the multimers in pharmaceutical preparations can be reduced to a level identical to or lower than those achieved by the conventional techniques and the present invention can thus provide a highly safe human serum albumin-containing pharmaceutical preparation.

According to the first method of the present invention, the multimers of human serum albumin can be converted into the monomers thereof and the monomers can be recovered simply by subjecting the multimers to an alkali-treatment and therefore, the method never suffers from any loss of human serum albumin unlike the conventional methods in which the multimers are removed. This indicates that the method of the invention permits the recovery of human serum albumin monomers in a high yield. Accordingly, the method of the present invention would permit the substantial reduction of the production cost of human serum albumin.

The second method of the present invention permits the efficient inhibition of any intra-molecular or inter-molecular uncorrected folding of human serum albumin, which is specifically caused in an oxidative atmosphere during the process for preparing HSA from the plasma or the process for preparing rHSA according to the gene recombination technique.

Moreover, the method of the present invention permits the preparation of high purity human serum albumin having a reduced content of a novel substance, which is formed through the uncorrected folding of human serum albumin and becomes a cause of side effects such as allergy observed when it is administered to a patient.

## Claims

1. A method for converting a multimer of human serum albumin into monomers thereof wherein the multimer is treated with an alkaline solution.

2. A method according to claim 1 wherein the multimer is treated with an alkaline solution in the presence of an SH group-containing compound.

3. A method for preventing the uncorrected folding of human serum albumin during a process for converting a multimer of human serum albumin into monomers thereof by treating the multimer with an alkaline solution, wherein the human serum albumin-containing solution is treated with the alkaline solution in the presence of an SH group-containing compound.

4. A method according to claim 2 or 3 wherein the concentration of the SH group-containing compound is from 0.1 to 50 mM or from 0.2 to 15 mM or from 0.5 to 5 mM.

5. A method according to any of claims 2 to 4 wherein the SH group-containing compound is selected from low molecular compounds including cysteine, cysteamine, cystamine and methionine.

6. A method according to any preceding claim wherein the human serum albumin is recombinant human serum albumin produced by gene recombination.

7. A method according to any preceding claim wherein the pH value of the alkaline solution is from 8 to 9.5 or from 8.5 to 9.5 or is 9.0.

8. A method according to any preceding claim wherein the treatment with the alkaline solution is carried out for not less than 15 minutes or for from 2 to 8 hours or for from 3 to 4 hours.

9. A method according to any preceding claim wherein the treatment with the alkaline solution is carried out at a temperature of from 0 to 65°C or at room temperature.

10. A method according to any preceding claim wherein the alkaline solution is prepared from a substance selected from alkaline organic and alkaline inorganic compounds including ammonia, ammonium salts, basic metal hydroxides, borates, phosphates, acetates, oxalates, citrates, tris-hydroxyaminomethane and mixtures of at least two of these substances.

11. A method according to claim 10 wherein the alkaline solution is prepared from a substance selected from ammonia, sodium hydroxide, potassium hydroxide, boric acid, borates, tris-hydroxyaminomethane and mixtures of at least two of these substances.

## Patentansprüche

1. Verfahren zum Umwandeln eines Multimers des humanen Serumalbumins in Monomere davon, wobei das Multimer mit einer alkalischen Lösung mit einem pH-Wert von 8 bis 9,5 behandelt wird.

2. Verfahren nach Anspruch 1, wobei das Multimer mit einer alkalischen Lösung mit einem pH-Wert von 8 bis 9,5 in Anwesenheit einer eine SH-Gruppe enthaltenden Verbindung behandelt wird.

3. Verfahren zum Verhindern von unkorrigiertem Falten des humanem Serumalbumins während des Verfahrens zum Umwandeln eines Multimers von humanem Serumalbumin in Monomere davon durch Behandeln des Multimers mit einer alkalischen Lösung mit einem pH-Wert von 8 bis 9,5, wobei die das humane Serumalbumin enthaltende Lösung mit einer alkalischen Lösung in Anwesenheit einer eine SH-Gruppe enthaltenden Verbindung behandelt wird.

4. Verfahren nach Anspruch 2 oder 3, wobei die Konzentration der eine SH-Gruppe enthaltenden Verbindung von 0,1 bis 50 mM oder von 0,2 bis 15 mM oder von 0,5 bis 5 mM ist.

5. Verfahren nach einem der Ansprüche 2 bis 4, wobei die eine SH-Gruppe enthaltende Verbindung aus niedermolekularen Verbindungen, einschließlich Cystein, Cysteamin, Cystamin und Methionin, ausgewählt wird.

6. Verfahren nach einem vorhergehenden Anspruch, wobei das humane Serumalbumin durch Genrekombination hergestelltes, rekombinantes humanes Serumalbumin ist.

7. Verfahren nach einem vorhergehenden Anspruch, wobei der pH-Wert der alkalischen Lösung von 8 bis 9,5 oder von 8,5 bis 9,5 oder 9 ist.

8. Verfahren nach einem vorhergehenden Anspruch, wobei die Behandlung mit der alkalischen Lösung für nicht weniger als 15 Minuten oder für 2 bis 8 Stunden oder für 3 bis 4 Stunden ausgeführt wird.

9. Verfahren nach einem vorhergehenden Anspruch, wobei die Behandlung mit der alkalischen Lösung bei einer Temperatur von 0°C bis 65°C oder bei Raumtemperatur ausgeführt wird.

10. Verfahren nach einem vorhergehenden Anspruch, wobei die alkalische Lösung aus einer Substanz, ausgewählt aus alkalischen organischen und alkalischen anorganischen Verbindungen, einschließlich Ammoniak, Ammoniumsalze, basische Metallhydroxide, Borate, Phosphate, Acetate, Oxalate, Citrate, Tris-Hydroxyaminomethan und Gemischen von mindestens zwei dieser Substanzen, hergestellt wird,

11. Verfahren nach Anspruch 10, wobei die alkalische, Lösung aus einer Substanz, ausgewählt aus Ammoniak, Natriumhydroxid, Kaliumhydroxid, Borsäure, Boraten, Tris-Hydroxyaminomethan und Gemischen aus mindestens zwei dieser Substanzen, hergestellt wird.

## Revendications

1. Procédé pour convertir un multimère de sérumalbumine humaine en ses monomères où le multimère est traité avec une solution alcaline de pH 8 à 9,5.

2. Procédé selon la revendication 1 où le multimère est traité avec une solution alcaline de pH 8 à 9,5 en présence d'un composé contenant un groupe SH.

3. Procédé pour empêcher l'assemblage non corrigé de la sérumalbumine humaine pendant un processus pour convertir un multimère de sérumalbumine humaine en ses monomères par traitement du multimère avec une solution alcaline de pH 8 à 9,5, où la solution contenant la sérumalbumine humaine est traitée avec la solution alcaline en présence d'un composé contenant un groupe SH.

4. Procédé selon la revendication 2 ou 3 où la concentration du composé contenant un groupe SH est de 0,1 à 50 mM ou de 0,2 à 15 mM ou de 0,5 à 5 mM.

5. Procédé selon l'une quelconque des revendications 2 à 4 où le composé contenant un groupe SH est choisi parmi les composés de faible masse moléculaire incluant la cystéine, la cystéamine, la cystamine et la méthionine.

6. Procédé selon l'une quelconque des revendications précédentes où la sérumalbumine humaine est de la sérumalbumine humaine recombinante produite par recombinaison génique.

7. Procédé selon l'une quelconque des revendications précédentes où le pH de la solution alcaline est de 8 à 9,5 ou de 8,5 à 9,5 ou est 9,0.

8. Procédé selon l'une quelconque des revendications précédentes où le traitement avec la solution alcaline est réalisé pendant au moins 15 minutes ou pendant 2 à 8 heures ou pendant 3 à 4 heures.

9. Procédé selon l'une quelconque des revendications précédentes où le traitement avec la solution alcaline est réalisé à une température de 0 à 65°C ou à la température ambiante.

10. Procédé selon l'une quelconque des revendications précédentes où la solution alcaline est préparée à partir d'une substance choisie parmi les composés organiques alcalins et les composés inorganiques alcalins incluant l'ammoniac, les sels d'ammonium, les hydroxydes métalliques basiques, les borates, les phosphates, les acétates, les oxalates, les citrates, le tris-hydroxyaminométhane et les mélanges d'au moins deux de ces substances.

11. Procédé selon la revendication 10 où la solution alcaline est préparée à partir d'une substance choisie parmi l'ammoniac, l'hydroxyde de sodium, l'hydroxyde de potassium, l'acide borique, les borates, le tris-hydroxyaminométhane et les mélanges d'au moins deux de ces substances.
